# EUROPEAN PATENT APPLICATION

(11) **EP 4 322 177 A1**
(43) Date of publication of application: **14.02.2024**
(21) Application number: 22190309.9
(22) Date of filing: 12.08.2022
(51) Int. Cl.: G16H 50/20, G16H 50/30, G16H 40/63

(54) **METHOD FOR DETERMINING A FATIGUE VALUE OF A PERSON**

(71) Applicant: Universität Zürich, 8006 Zürich (CH); ETH Zurich, 8092 Zürich (CH)
(72) Inventor: Lutterotti, Andreas, 8006 Zürich (CH); Oldrati, Pietro, 8006 Zürich (CH); Amon, Rok, 8006 Zürich (CH); Hilty, Marc, 8006 Zürich (CH); Barrios, Liliana, 8006 Zürich (CH)
(74) Representative: Schulz Junghans Patentanwälte PartGmbB

(57) **Abstract**

The invention relates to a method, particularly a computer-implemented method for determining a fatigue value of a person, the method comprising the steps of:
i) displaying of:
a) a first group (1) of symbols and a second group (2) of symbols,
b) a selection rule (3) associating each of the symbols of the first group (1) of symbols to one symbol of the second group (2) of symbols,

ii) highlighting one symbol (11) of the first group (1) of symbols,
iii) receiving a user input from the person selecting one symbol (12) of the second group (2) of symbols,
iv) determining a truth value based on the selected symbol (12) of the second group (2) of symbols, wherein the truth value assumes a true value if the selected symbol (12) is selected in accordance with the selection rule (3) and wherein the truth value takes on a false value if not,
v) storing the truth value on a non-transitory memory device,
vi) changing the selection rule (3), such that each of the symbols of the first group (1) of symbols is associated anew to one symbol of the second group (2) of symbols,
vii) changing the highlighted symbol (11) of the first group (1) of symbols,
viii) repeating steps iii) to vii) at least once,
ix) based on at least some of the stored truth values, determining a fatigue value of the person.

The invention also relates to a system for determining a fatigue value of a person as well as a computer program for carrying out the method according to the invention.

## Description

The present invention relates to a method, particularly a computer-implemented method for determining a fatigue value of a person. The invention also relates to a computer program to partially carry out the method according to the invention. Moreover, the invention relates to a system for determining a fatigue value of a person.

Fatigue is a highly prevalent and devastating symptom of many diseases, including Parkinson's disease, Multiple Sclerosis (MS), and more recently, post-COVID syndrome. In MS, fatigue is rated as the most frequent and debilitating symptom [1,2,3]. Fatigue has been defined as the subjective feeling of overwhelming exhaustion and tiredness and can manifest as a physical and cognitive symptom [4]. The symptom is still poorly understood, and its severity can only be assessed subjectively. Currently, this is done using questionnaires such as the Fatigue Severity Scale (FSS) [5], Modified Fatigue Impact Scale (MFIS) [6], and Fatigue Scale for Motor and Cognitive Functions (FSMC) [7]. More than a dozen fatigue questionnaires are available [8]. These questionnaires are used as patient reported outcome measures in clinical trials. Their heterogeneity and subjective nature are a challenge for using them as outcome measures in clinical trials and comparing the efficacy of results across different studies. Results from different randomized placebo-controlled clinical trials testing different compounds for treating fatigue showed contradictory results, with some showing good efficacy, and others exhibiting no effect [9-16].

The perception of fatigue (subjective measurement) is being differentiated from performance fatigability (objective measurement) [17]. Fatigability is further divided into the motor and cognitive domains. Motor fatigability has been quantified as the decline in peak performance, power, or speed during physical activity [18]. On the other hand, cognitive fatigability measures the decline of cognitive performance during a task that requires sustained attention [18], and it has been measured as an increase in reaction time, decline in accuracy or by comparing the performance during the first and last third of a task [19,20]. Establishing an association between objective fatigability and subjective fatigue is an important goal for clinical research but has been proven difficult [17]. While a correlation between motor fatigability and perceived fatigue has been suggested in several studies [21-25], less data is available on cognitive fatigability [26-33]. A possible cause is the complexity of inducing cognitive fatigability and the lack of consensus and dedicated tests to quantify it [20]. Prior studies used one of two strategies to generate cognitive fatigability. Either they conducted a test battery, including the same test before and after fatiguing tasks and compared their performance, or they employed a single prolonged cognitive task and measured the decline in performance within the task. Some of the used cognitive tests within fatigability research include: (1) the Paced Auditory Serial Addition Test (PASAT) [34], (2) the Psychomotor vigilance task (PVT) [35], and (3) the Stroop test [36]. However, utilizing these non-specific cognitive performance tests to assess cognitive fatigability comes with certain drawbacks, such as long testing sessions.

Fatigability in healthy subjects is typically studied through long examination sessions. Van der Linden et al. induced fatigue through two hours of cognitively demanding tasks. Their study showed a significant difference in planning ability and increased perseverative errors between the non-fatigued and fatigued participants [30]. Other cognitive fatigability studies in healthy subjects using the Stroop test employed a study length of 3 and 2 hours for young adults [32] and for older adults [33], respectively. However, long testing sessions are not unique to healthy subjects. Moeller et al. administered two hourly test batteries for analyzing cognitive fatigability using three neuropsychological tests in subjects with mild traumatic brain injury [31]. In MS, there is large heterogeneity when it comes to studying cognitive fatigability. DeLuca et al. [37] studied fatigue in 15 persons with MS and 15 controls by conducting four modified SDMT (mSDMT) trials over an hour of fMRI scanning where patients were shown different symbol-digit pair probes at varying interstimulus. Participants had to respond "match" or "no match" to each probe by following a provided symbol-digit arrangement. The interstimulus interval randomly varied between 0, 4, 8 or 12 s. Results from their study found no cognitive fatigability. Chen et al. [38] also studied fatigability using a mSDMT within a fMRI setting. During examination people with MS and controls completed a total of eight mSDMT (4 with high cognitive load and 4 with low cognitive load), each lasting 7.7 minutes. The authors did not study within trial performance, but across trial performance showed an increase in reaction time associated with subjective fatigue in persons with MS. Berard et al. compared the performance during quintiles of a twenty-minute PVT session to quantify cognitive fatigability and found a greater increase in reaction time of patients compared to healthy controls [29]. PVT is a simple reaction time task where participants have to press a button in response to the presence of a stimulus. However, its repetitive and monotonous nature often results in participants reporting feelings of boredom [39], and thus the performance decline may be influenced by a lack of motivation rather than fatigability [40]. Finally, several authors employed the PASAT by comparing the decrease in accuracy between the beginning and end of the test [26-28], [40-42]. Even though the PASAT is applied in many studies, there is still significant methodological heterogeneity. First, some studies compared the performance between the first and the second half [26] of the test, while others compared the performance between thirds [27]. Second, despite there seems to be a general consensus of 3 seconds length interstimulus interval (ISI), this has not been uniformly applied in fatigability studies [18,28,42]. Third, it is known that persons with MS may adopt a 'chunking strategy,' particularly as task demands increase [43], meaning that they add two numbers, skip one, and add the following two, thus, reducing the overall difficulty of the task by decreasing the simultaneous cognitive load. Only recently, first normative data on cognitive fatigability has been generated to account for the chunking strategy [41]. Fourth, the PASAT requires a medical examiner to conduct the test, making it more expensive to administer. Finally, patients have described the PASAT as unpleasant and causing anxiety [44], limiting the applicability and repeatability of the tests.

A reliable and objective measurement of fatigability would help quantify the effectiveness of treatments, both in clinical trials and routine care, and it would also help clinicians distinguish between confounding comorbidities. Several randomized placebo-controlled clinical trials tested different compounds for treating fatigue [9-16]. However, results from these clinical trials are inconsistent. A common denominator in these trials is that they quantified the outcome measure using subjective questionnaires. It is known that the magnitude of the placebo effect is an important reason for the variability in the efficacy during trials [14, 45,46]. Thus, an objective measurement would help clinicians overcome these limitations and complement questionnaires to evaluate treatments' efficacy.

Based on this, it is subject of the present invention to provide a method, particularly a computer-implemented method, a computer program as well as a system for determining a fatigue value of a person which allows for a more objective determination of the fatigue of the person.

This task is solved by a method for determining a fatigue value of a person with the features of claim 1, a computer program for determining a fatigue value of a person with the features of claim 10 as well as a system for determining a fatigue value of a person with the features of claim 11.

Advantageous embodiments of the invention are given in the corresponding subclaims and described in the following.

A first aspect of the invention relates to a method, particularly a computer-implemented method for determining a fatigue value of a person. The method comprises the following steps:
i) displaying of:
   a) a first group of symbols and a second group of symbols,
   b) a selection rule associating each of the symbols of the first group of symbols to one symbol of the second group of symbols,
ii) highlighting one symbol of the first group of symbols
iii) receiving a user input from the person selecting one symbol of the second group of symbols,
iv) determining a truth value based on the selected symbol of the second group of symbols, wherein the truth value assumes a true value if the selected symbol is selected in accordance with the selection rule and wherein the truth value takes on a false value if not,
v) storing the truth value on a non-transitory memory device,
vi) changing the selection rule and particularly displaying the changed selection rule, such that each of the symbols of the first group of symbols is associated anew to one symbol of the second group of symbols,
vii) changing the highlighted symbol of the first group of symbols
viii) repeating steps iii) to vii) at least once and
ix) based on at least some of the stored truth values, determining a fatigue value of the person.

For example, the symbols may be geometrical symbols comprising geometrical shapes such as for example circles, curved elements and/or polygons, particularly sections of circles, curved elements and/or polygons. Particularly, the symbols may comprise multiple geometrical symbols. The symbols may also comprise images, colors, numbers, digits, letters or special characters.

Preferably, the first group of symbols comprises symbols that are distinct from the symbols of the second group of symbols, such that the person may link symbols from two distinct groups, which provides a clear selection rule based on two groups of symbols with distinct symbols.

The first and the second group of symbols, the selection rule as well as the highlighted symbol of the first group may for example be displayed to the person by a screen. The screen can be part of a portable device, such as a tablet or smartphone, but it can also be part of a stationary device such as a stationary screen connected to a stationary computer. The displaying may also be done by a projecting device or any other means configured to provide the person with the first and the second group of symbols, the selection rule as well as the highlighted symbol of the first group.

Preferably, the selection rule establishes an unambiguous connection between a respective symbol of the first group of symbols and an associated symbol of the second group of symbols and vice versa. To this end, the symbols of the first group of symbols may for example be displayed in proximity to their respective associated symbols of the second group of symbols, such that the unambiguous connection between each symbol of the first group and its respective associated symbol of the second group becomes clear to the person. For example, each symbol of the first group of symbols may be displayed closer to its respective associated symbol of the second group of symbols compared to the other symbols of the second group of symbols. The selection rule may also be displayed to the person by means of interconnects such as for example lines or arrows between each symbol of the first group of symbols and their respective associated symbol.

To highlight one symbol of the first group according to step ii), one symbol of the first group is preferably displayed in a representation that is distinct from the other symbols of the first group. For example, to this end, one symbol of the first group may be highlighted by displaying it bigger or smaller, more or less bold or in a different color compared to the other symbols of the first group. Alternatively, the one symbol of the first group may also be highlighted by displaying it separately, that is displaying it at least twice, wherein the other symbols of the first group are only displayed once, respectively. The highlighting may also comprise additional symbols, for example by means of underscoring or framing one symbol of the first group.

The term "receiving a user input" particularly relates to receiving a user input from the person via a user interface.

With respect to step iii), the selection of one symbol of the second group of symbols by the person is preferably executed by means of a user input from the person. For example, to this end, a user interface can be provided, wherein the person can interact with the user interface so as to select symbols of the second group of symbols. For instance, the interface can be part of a portable device. In particular, the selection may be performed on a touchscreen, wherein the touchscreen may at the same time be configured to display the symbols and the highlighted symbols according to steps i) and ii) of the method according to the invention. Alternatively, the selection of symbols may be performed on an interface which is separate from the screen or an interface for displaying the symbols and the highlighted symbols. The user input may also comprise vocal inputs of the users and the interface may be configured for voice recognition.

Regarding step iv), the truth value is preferably a binary value that assumes one of two possible values, such as for example 0 or 1; yes or no; true or false. For a given highlighted symbol of the first group, the truth value may be determined particularly by a processing unit by comparing the symbol of the second group of symbols based on the user input of the person in step iii) to the symbol of the second group which is associated to the highlighted symbol of the first group. Particularly, if the selected symbol coincides with the logically associated symbol, the truth value assumes one of the two values, wherein if the selected symbol does not coincide with the logically associated symbol, the truth value assumes the other of the two values.

Upon the determination of the truth value based on the symbol selected by the person, according to step v), the truth value is stored on a non-transitory memory device. Subsequently, in step vi), the selection rule is changed and particularly displayed to the user such that each of the symbols of the first group of symbols I associated anew to one symbol of the second group of symbols and in step vii), the highlighted symbol of the first group of symbols is changed and particularly displayed to the user.

Based on the changed and particularly displayed selection rule and highlighted symbol, the person subsequently provides further user input by selecting symbols of the second group of symbols, wherein the further user input is also stored on the non-transitory memory device. In other words, the steps iii) to vii) of the method according to the invention are repeated at least once.

Particularly, the highlighted symbol and the selection rule may be changed according to a randomization process. As such, after every selection of a symbol, the person is confronted with a changed, particularly a new highlighted symbol and a changed, particularly a new selection rule, such that no learning effects can occur. As a consequence, the phenomenon of fatigue may be studied without artificial side effects corresponding to a learning or habituation effects.

In step ix), the fatigue value is determined based on at least some of the stored truth values.

Particularly, only truth values that have been determined within an examination phase of the method for determining a fatigue value of a person are considered for the determination of the fatigue value.

According to another embodiment, the highlighted symbol of the first group of symbols is highlighted for a predetermined time, particularly a predetermined time based on a statistical response time of the person, and wherein if the user input is not received within the predetermined time in step iii), the method is set forth with step iv), having the truth value set to the false value. Particularly, this embodiment may be part of the examination phase.

Particularly, the statistical response time is determined from time intervals between the displaying of a highlighted symbol of the first group of symbols and the selection of a symbol of the second group of symbols by the person.

Said predetermined time may thus form a time limit for the person to select a symbol of the second group upon displaying of a highlighted symbol of the first group serving as a motivation for the person to select a symbol before exceeding the time limit, which would result in a false value of the truth value generated for the determination of the fatigue value. Particularly, once the time limit is exceeded, the highlighted symbol of the first group of symbols and the selection rule is changed and the time limit is reset and particularly reapplied, such that the person may repeatedly select symbols of the second group of symbols within the time limit, wherein if the person does not select symbols, the time limit is reset multiple times.

In particular, the predetermined time or time limit may be based on a statistical response time of the person determined within a calibration phase prior to the examination phase of the method for determining a fatigue value of a person.

Particularly, the selection of symbols of the second group of symbols by the person is restricted to a predetermined examination period. For example, the predetermined examination period may be between 5 minutes and 30 minutes or between 2.5 minutes and 7.5 minutes, particularly around 5 minutes.

According to an embodiment, the fatigue value is computed by means of a relative change between a first number of true and/or false values determined within a first section of the predetermined examination period and a second number of true and/or false values determined within a second section of the predetermined examination period, wherein the second section is spaced apart in time from the first section.

Alternatively or additionally, the fatigue value is computed by means of a relative change between a first statistical response time determined within the first section of the predetermined examination period and a second statistical response time determined within the second section of the predetermined examination period.

Particularly, the first and the second section are of identical length in time. For example, the first section may be the first third of the examination period and the second section may be the last third of the examination period.

As such, the fatigue value directly reflects a trend of true or false values and/or a trend of the statistical response time as a function of time elapsed during the examination period, representing a direct measure of fatigue phenomena.

According to another embodiment, prior to the examination phase, a preparation phase is conducted, wherein during the preparation phase, steps i) to viii) are executed wherein upon every selection of a symbol of the second group of symbols by the person, a signal indicative of the truth value of the selected symbol is displayed to the person. Preferably, during the preparation phase, the determined truth values are not considered for the determination of the fatigue value.

Particularly, the signal may be displayed to the person within a preparation phase. As such, the person may obtain direct feedback about the truth value of every selected symbol, which helps the person to become familiar with the method.

According to this embodiment, the selection of symbols of the second group of symbols by a person can be restricted to a predetermined preparation period of the preparation phase. For example, the preparation phase may be constricted to a preparation period between one minute and three minutes, particularly approximately 2 minutes.

According to another embodiment, between the preparation phase and the examination phase, a calibration phase is conducted, wherein during the calibration phase:
- steps i) to viii) are executed,
- the selection of symbols of the second group of symbols by the person is restricted to a predetermined calibration period and
- the statistical response time is determined, wherein the statistical response time is determined from time intervals between the displaying of a highlighted symbol of the first group of symbols and the selection of a symbol of the second group of symbols by the person.

Particularly, during the calibration phase, the signal indicative of the truth value of the selected symbol is not displayed to the person, such that the person is no longer provided with feedback about the truth value based on the selected symbols.

The statistical response time may for example be or comprise an average time calculated by means of the average of at least some of, particularly all determined time intervals between the displaying of a highlighted symbol of the first group of symbols and the selection of a symbol of the second group of symbols by the person. Particularly, the statistical response time may be a percentile of the response time, for example between the 80^{th} and the 90^{th}, and particularly the 85^{th} percentile of the response time.

Particularly, the statistical response time determined during the calibration phase may be used as the limit for the person to select a symbol of the second group of symbols during the examination phase. Hence, the calibration phase allows for a normalization of the time limit, adapting or tailoring it to the mental and/or physical fitness of the person. For instance, if a first statistical response time is determined for a first person and a second statistical response time is determined for a second person, wherein the first statistical response time is lower than the second statistical response time, the predetermined time or time limit in the examination phase may be adapted individually for both persons, such that the during the examination phase, fatigue phenomena can be studied independently of the states of mental and/or physical fitness of the person.

For example, the predetermined calibration phase may be restricted to a calibration period between 30s and 90s, particularly approximately 60s.

Particularly, the preparation phase may be conducted prior to the examination phase.

In yet another embodiment, the method according to claim 8 is only performed if during the method according to claim 7, the person has selected a predetermined minimum number of symbols of the second group of symbols and if a predetermined minimum ratio between a number of true values determined based on the selected symbols and the minimum number of symbols has been determined.

For example, the predetermined minimum number of selected symbols of the second group of symbols is between 10 and 30, particularly around 20. For example, the predetermined minimum ratio may be between 0.6 and 0.8, particularly around 0.7, meaning that between 60% and 80%, particularly around 70% of all selected symbols have been selected in accordance with the respective selection rule.

As such, the calibration phase may only be started once the person has understood the principle of the selection and the selection rule by implementing said predetermined minimum number of symbols and said predetermined minimum ratio as a condition to advance to the calibration phase.

A second aspect of the invention relates to a computer program comprising instructions which, when the program is executed by a processing unit, cause the processing unit to carry out method according to one of the preceding claims.

According to an embodiment, the computer program comprises instructions which, when the program is executed by the processing unit, cause the processing unit to highlight the highlighted symbol of the first group of symbols for a predetermined time, particularly a predetermined time based on a statistical response time of the person, and wherein if the user input is not received within the predetermined time in step iii) of the method, the computer program causes the processing unit to set the method forth with step iv) having the truth value set to the false value.

According to an embodiment, the computer program comprises instructions which, when the program is executed by the processing unit, cause the processing unit to restrict the selection of symbols of the second group of symbols by the person during an examination phase to a predetermined examination period having a first and a second section, wherein the first section is spaced apart in time from the second section.

According to an embodiment, the computer program comprises instructions which, when the program is executed by the processing unit, cause the processing unit to during the examination phase, compute the fatigue value by means of a relative change between a first number of stored true and/or false values determined within the first section of the predetermined examination period and a second number of stored true and/or false values determined within the second section of the predetermined examination period.

According to an embodiment, the computer program comprises instructions which, when the program is executed by the processing unit, cause the processing unit to during the examination phase, compute the fatigue value by means of a relative change between a first statistical response time determined within the first section of the predetermined examination period and a second statistical response time determined within the second section of the predetermined examination period.

According to an embodiment, the computer program comprises instructions which, when the program is executed by the processing unit, cause the processing unit to conduct or execute a preparation phase prior to the examination phase, wherein during the preparation phase, steps i) to viii) of the method according to the invention are executed based on instructions of the computer program, wherein upon every selection of a symbol of the second group of symbols by the person, a signal indicative of the truth value of the selected symbol is displayed to the person.

According to an embodiment, the computer program comprises instructions which, when the program is executed by the processing unit, cause the processing unit to restrict the selection of symbols of the second group of symbols by the person to a predetermined preparation period of the preparation phase.

According to an embodiment, the computer program comprises instructions which, when the program is executed by the processing unit, cause the processing unit to conduct or execute a calibration phase between the preparation phase and the examination phase, wherein during the calibration phase:
- steps i) to viii) of the method are executed based on instructions of the computer program
- the selection of symbols of the second group of symbols by the person is restricted to a predetermined calibration period, and
- the statistical response time is determined, wherein the statistical response time is determined from time intervals between the displaying of a highlighted symbol (11) of the first group (1) of symbols and the selection of a symbol of the second group (2) of symbols by the person.

According to an embodiment, the computer program comprises instructions which, when the program is executed by the processing unit, cause the processing unit to only perform the method according to claim 8 if during the method according to claim 7, the person has selected a predetermined minimum number of symbols of the second group of symbols and if a predetermined minimum ratio between
- a number of true values determined based on the selected symbols and
- the minimum number of symbols
has been determined.

Particularly, the processing unit may be part of a portable device such as a smartphone or tablet, such that for determination of a fatigue value of a person does not require qualified staff or require the person to approach a physician or a hospital, but may be done remotely like for example at home.

A third aspect of the invention relates to a system for determining a fatigue value of a person, wherein the system comprises:
- a display configured to display:
   a) a first group of symbols and a second group of symbols,
   b) a selection rule logically associating each of the symbols of the first group of symbols to a respective associated symbol of the second group of symbols,
   wherein one symbol of the first group of symbols is highlighted,
- a user interface for receiving a user input from the person, wherein the user input is indicative of selections of symbols of the second group of symbols by the person and
- a processing unit, particularly configured to execute the computer program of claim 10, wherein the processing unit is configured to
   i) determine truth values based on the selected symbols of the second group of symbols, wherein the truth value assumes a true value if the selected symbol of the second group of symbols was selected in accordance with the selection rule and wherein the truth value assumes a false value if not
   ii) store the truth values in a non-transitory memory device
   iii) change the selection rule upon every selection of a symbol of the second group of symbols by the person such that each of the symbols of the first group of symbols is associated anew to one symbol of the second group of symbols,
   iv) change the highlighted symbol of the first group of symbols upon every selection of a symbol of the second group of symbols by the person and
   v) determine a fatigue value of the person based on at least some of the stored truth values.

Particularly, the system is configured to execute the method according to the first aspect of the invention.

Particularly, the processing unit of the system according to the third aspect of the invention is configured to execute the method according to the second aspect of the invention.

In another embodiment, the system is configured to set a time limit for the person to select a symbol of the second group of symbols and to cause said display to display a time indicator indicative of a time left until said time limit is reached and wherein the system is further configured to change said highlighted symbol of the first group of symbols and said selection rule if the person does not select a symbol of the second group of symbols within said time limit. Particularly, the time limit is based on a statistical response time based on time intervals between the displaying of a highlighted symbol of the first group of symbols and the selection of a symbol of the second group of symbols by the person.

Particularly, the system is configured to determine a statistical response time of the person determined from time intervals between the displaying of a highlighted symbol of the first group of symbols and the selection a symbol of the second group of symbols by the person, wherein the time limit is based on the statistical response time of the person.

According to an embodiment, the system is further configured to set the truth value to the false value if the person does not select a symbol of the second group of symbols within the time limit.

In yet another embodiment, the processing unit is configured to set a predetermined examination period for selection of symbols of the second group of symbols by the person and wherein the processing unit is further configured to determine and particularly to output the fatigue value based on a relative change of a first number of true or false values determined within a first section of the predetermined examination period and a second number of true or false values determined within a second section of the predetermined examination period, wherein the second section is spaced apart in time from the first section; and/or based on a relative change of a first statistical response time determined within the first section of the predetermined examination period and a second statistical response time determined within the second section of the predetermined examination period.

Exemplary embodiments are described below in conjunction with the Figures. The Figures are appended to the claims and are accompanied by text explaining individual features of the shown embodiments and aspects of the present invention. Each individual feature shown in the Figures and/or mentioned in the text of the Figures may be incorporated (also in an isolated fashion) into a claim relating to the method, the computer program and the system for determining a fatigue value of a person according to the present invention.
- Fig. 1: shows a display with a user interface for executing the method for determining a fatigue value of a person;
- Figs. 2a,b: show another user interface wherein a correct (a) and a wrong (b) truth value is output by means of a signal indicating the truth value of a selected symbol of the second group of symbols for a given highlighted symbol of the first group of symbols;
- Fig. 3: depicts a preparation phase and a calibration phase of the method, wherein the calibration phase is only performed if during the preparation phase, the person has selected a minimum number of symbols of the second group of symbols and if a predetermined minimum ratio between a number of true values determined based on the selected symbols and the minimum number of symbols has been determined;
- Figs. 4a,b: shows an exemplary user interface that may particularly be used for the examination phase of the method for determining a fatigue value of a person, with a time indicator indicating a time left for selection of a symbol of the second group of symbols until the highlighted symbol of the first group of symbols is changed, wherein in Fig. 4b less time is left than in Fig. 4a;
- Fig. 5: shows an example embodiment of the method according to the invention, wherein the examination phase is executed after the preparation phase and the calibration phase according to Fig. 3;
- Fig. 6: shows measured response times between the displaying of a highlighted symbol of the first groups of symbols and a selection of a selected symbol of the second group of users by a person before (left) and after (right) removal of artifacts;
- Fig. 7: depicts measured normalized averaged response times over segments of 30 seconds during a five-minute examination period;
- Fig. 8: shows a flow chart of a study conducted using an embodiment of the method according to the invention;
- Fig. 9: shows measured response times averaged over a first, second and third third of the examination period for non-fatigued persons with multiple sclerosis (left) and for fatigued persons with multiple sclerosis (right);
- Fig. 10: depicts mean AUROC data for Δresponse time (ART) generated using a Monte-Carlos simulation with 1000 iterations (left) and t-test results for Δresponse time (center, right) and average response time (right).
- Fig. 11: shows mean AUROC data for average response time (RT) and Δresponse time (ART) (left), t-test results for Δresponse time (center) and average response time (right).

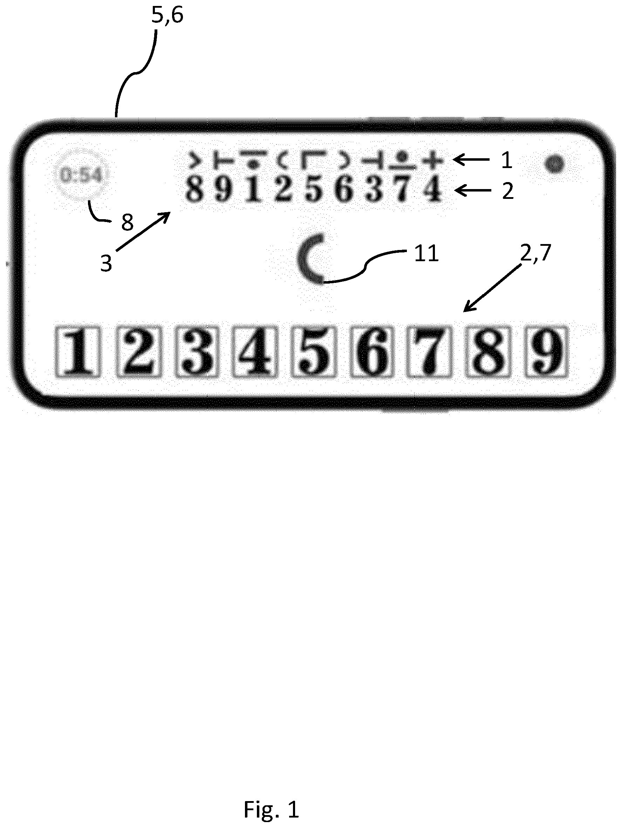

Fig. 1 shows a display 5 with a user interface 6 for executing the method for determining a fatigue value of a person. Particularly, the display 5 may be part of a portable device such as a smartphone or a tablet. The portable device may also comprise a processing unit, such that the method may be performed by executing the computer program according to the second aspect of the invention on said processing unit.

In this example, the display 5 displays a first group 1 of symbols comprising nine different symbols comprising different geometrical shapes in an upper section of the display 5. Below the first group 1 of symbols, the display 5 also shows a second group 2 of symbols comprising nine different digits from one to nine. Every symbol of the first group 1 of symbols is thereby arranged next to a respective symbol of the second group 2 of symbols, such that each of the symbols of the first group 1 of symbols is logically associated to a respective associated symbol of the second group 2 of symbols. As such, this logical association between the symbols of the first and the second group 1,2 of symbols represents a selection rule 3.

As can further be seen in Fig. 1, the display 5 further highlights a highlighted symbol 11 of the first group 1 by displaying it a second time as a magnified copy in the center of the display 5. Moreover, in a lower section of the display 5, the display 5 shows a copy of the second group 2 of symbols forming a selection panel 7. By interacting with the selection panel 7, the person may select a symbol of the second group 2 of symbols. Particularly, the display 5 may be a touchscreen such the display 5 may output information generated by the processing unit to the person by displaying the displayed symbols as described above but it may at the same time serve as an input, forwarding information comprising the interaction of the person with the display 5 to the processing unit, particularly information about the selected symbol of the second group 2 of symbols.

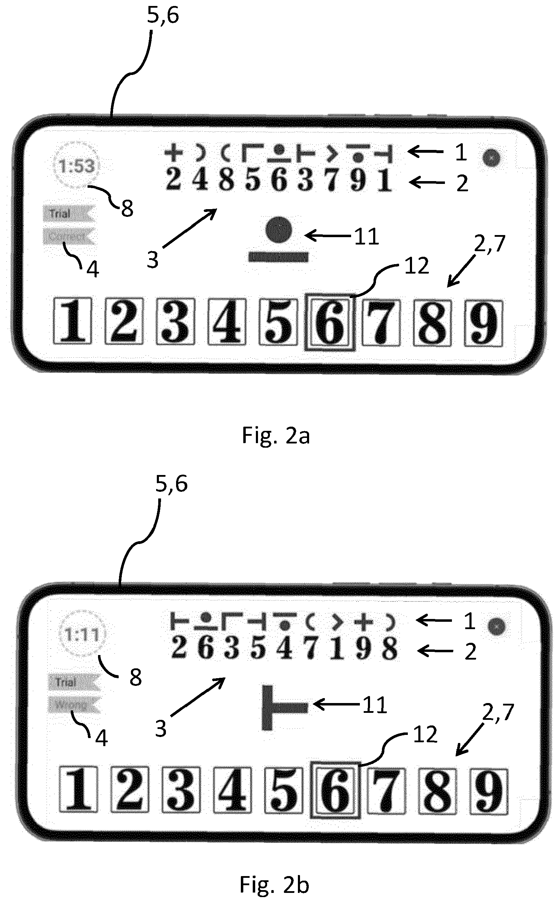

Figs. 2a,b show another example with a user interface 6 displayed on a display 5, wherein in Fig. 2a, a first selection rule 3 is displayed and wherein a first highlighted symbol 11 of the first group 1 of symbols is highlighted. As can be seen in the selection panel 7, one symbol of the second group 2 of symbols is framed, indicating a selected symbol 12 which has been selected by a person by interacting with the user interface 6. As can be understood by comparing the selected symbol 12 and the highlighted symbol 11 to the selection rule 3 in Fig. 2a, the selected symbol 12 has been selected in accordance with the selection rule 3. In this example, the user interface 6 is configured to output a signal 4 indicative of a truth value based on the selected symbol 12 of the second group 2 of symbols. Since in Fig. 2a, the selected symbol 12 has been selected in accordance with the selection rule 3, the signal 4 shows 'correct'.

Fig. 2b, shows a second selection rule 3 and a second highlighted symbol 11 which differ from the first selection rule 3 and the first highlighted symbol 11 shown in Fig. 2a. Particularly, every time the person selects a symbol of the second group 2 of symbols, the highlighted symbol 11 of the first group 1 of symbols is changed and the selection rule 3 is changed, such that each of the symbols of the first group 1 of symbols is associated anew to a respective associated symbol of the second group 2 of symbols. As can be understood by comparing the selected symbol 12 and the highlighted symbol 11 to the selection rule 3 in Fig. 2b, the selected symbol 12 has not been selected in accordance with the selection rule 3. The signal 4 therefore shows 'wrong', indicating that the selected symbol 12 has not been selected in accordance with the selection rule 3.

As such, in this example, the person obtains direct feedback about the truth value of every selected symbol via the user interface 6, which helps the person to become familiar with the method for determining a fatigue value of the person. This user interface 6 may therefore particularly be used for a preparation phase conducted prior to an examination phase of the method according to the invention. The motivation for providing immediate feedback is to help the person understand the principle of the selection of symbols according to the selection rule 3. This functionality is particularly beneficial for unsupervised settings where no medical examiner is present to clarify doubts to the patients.

As can be further seen in Figs. 2a,b, the user interface 6 also shows a timer panel 8. The timer panel 8 may indicate a time left for the person to select symbols of the second group 2 of symbols. As such, the preparation phase may be limited to a predetermined preparation period. For example, the preparation period may be between one minute and three minutes, particularly approximately 2 minutes.

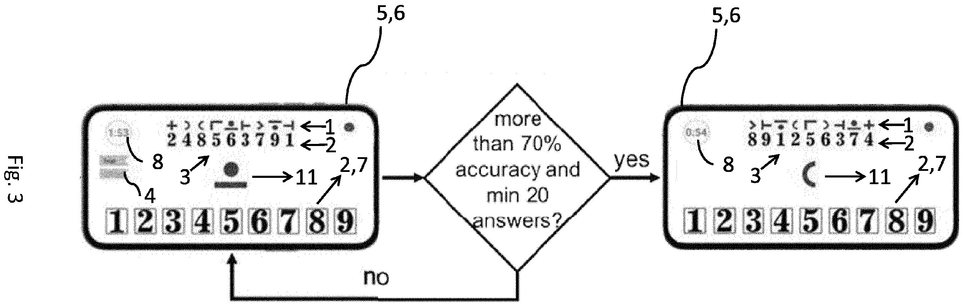

Fig. 3 shows another example in which a calibration phase of the method is conducted only if during the preparation phase before the calibration phase, the person has selected a predetermined number of symbols of the second group 2 of symbols and if based on the selected symbols 12, a minimum number of true values have been determined based on the selected symbols 12. Particularly, the calibration phase may be only conducted if a predetermined ratio between the minimum number of true values and the number of selected symbols of the second group 2 of symbols has been determined. For example, as indicated in Fig. 3, the calibration phase may only be conducted once the person has selected at least twenty symbols of the second group 2 of symbols, wherein for more than 70% of the selected symbols 12, a true truth value has been determined. As such, the person may first have the opportunity to understand the principle of the method for determining a fatigue value of the person during the preparation phase, before the calibration phase is conducted.

Particularly, within the calibration phase, the user interface 6 displayed on the display 5 does no longer show the signal 4 indicative of the truth value of the selected symbol, such that during the calibration phase, the person is no longer provided with a feedback about the truth value of the selected symbols.

The calibration phase may be restricted to a calibration period of for example between one minute and 10 minutes or between 30s and 90s, particularly approximately 60s. During the calibration phase, a statistical response time may be determined based on time intervals between the displaying of a highlighted symbol 11 of the first group 1 of symbols and the selection of a symbol of the second group 2 of symbols by the person. For example, the statistical response time may comprise or be an average time calculated by means of the average of at least some of, particularly all determined time intervals. Particularly, the statistical response time may be a percentile of the response time, for example between the 80^{th} and the 90^{th}, and particularly the 85^{th} percentile of the response time. The statistical response time may further be used during the examination phase, which is preferably conducted after the calibration phase. As such, each individual person may provide a user input according to step iii) of the method according to the invention at different rates but always in relation to their top performance. Thus, the statistical response time is tailored to each person, accounting for patients' different levels of disability.

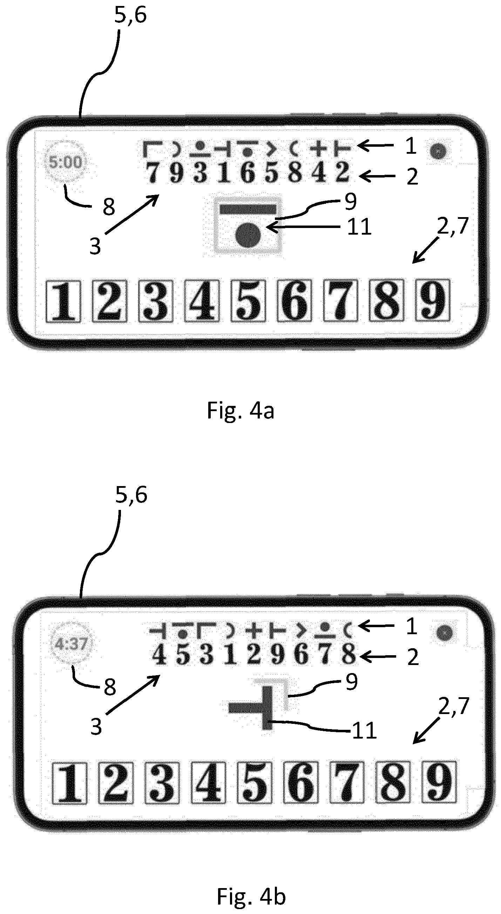

Figs. 4a,b show example user interfaces 6 that may particularly be used for the examination phase of the method, wherein a fatigue value of the person is determined. In particular, the user interfaces 6 depicted here comprise a time indicator 9 indicating a predetermined time or time limit indicative of a time left for a person to select a symbol of the second group 2 of symbols. For example, in Fig. 4a, the time indicator 9 forms a full square, indicating that the person has the full time limit left for selecting a symbol of the second group 2 of symbols. In contrast, as can be seen in Fig. 4b, the time indicator 9 forms only a section of the full square from Fig. 4a, indicating that time has elapsed and only a fraction of the time limit is left for the person to select a symbol of the second group 2 of symbols. Particularly, the time indicator 9 may be indicate the time left for a person to select a symbol in a linear manner, i.e. such that the size or extent of the remaining time indicator 9 scales linearly with the time left to select a symbol, wherein if the time indicator 9 has a maximum size, the time limit is full and wherein if the time indicator 9 has completely disappeared, the time limit is zero. If the person does not select a symbol of the second group 2 of symbols within the time limit, a false value of the truth value is generated. Particularly, once the time limit is reached, the highlighted symbol 11 of the first group 1 of symbols and the selection rule 3 is changed and the time limit is reset and particularly reapplied, such that the person may repeatedly select symbols of the second group 2 of symbols within the time limit, wherein if the person does not select symbols, the time limit is reset multiple times. As such, the time limit serves as a motivation for the person to select symbols within the time limit. Moreover, the added pressure to make a selection within the time limit contributes to the cognitive load required to induce cognitive fatigability.

Particularly, the predetermined time or time limit may be based on the statistical response time obtained within the calibration phase prior to the examination phase.

As can further be seen in Figs. 4a,b an exit button 10 is located in the top right corner of the user interface 6 shown on the display 5. By interacting with, particularly by selecting or pressing the exit button 10, the person can stop and exit the examination phase at any time. Preferably, if the person stops the test before the end of the examination period, the examination phase is considered invalid and no fatigue value is determined. The exit button 10 may also be applied to stop and exit the preparation phase and the calibration phase, respectively.

The examination phase is preferably limited to a predetermined examination period, which may for example be between 5 minutes and 30 minutes or between 2.5 minutes and 7.5 minutes, particularly around 5 minutes.

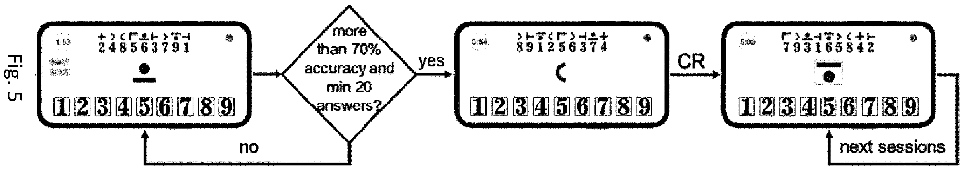

Fig. 5 shows an example embodiment of the method according to the invention, wherein first a preparation phase, for example as according to Figs. 2a,b or Fig. 3 is executed, such that the person becomes familiar with the principle of the displaying and selection of symbols according to the selection rule 3. In a subsequent calibration phase, for example as according to Fig. 3, a statistical response time of the person may be determined. The statistical response time determined in the calibration phase may then be used for the predetermined time or time limit for the person to select symbols during the examination phase to be executed after the calibration phase. The fatigue value is preferably determined based on truth values determined only during the examination phase, so as to minimize artefacts due to misunderstandings or confusion of the person.

As such, this embodiment and in particular the examination phase differs from the symbol digit modalities test (SDMT) known from the prior art in several aspects:
- The present embodiment and in particular the examination phase provide a cognitive fatigability test, while SDMT assesses cognitive impairment and working memory.
- Contrary to the SDMT, the present embodiment and in particular the examination phase does not allow the person to look ahead to match the following symbols based on the selection rule 3. Hence, the person has no way to anticipate the next selection to reduce the statistical response time.
- After a selection of a symbol or after exceeding the time limit, the selection rule 3 is changed, particularly randomized, while SDMT has a fixed selection rule 3.
- The examination period is preferably around 5 minutes, while the SDMT lasts 90 seconds. The increased duration is preferred because cognitive fatigability is notoriously hard to elicit in a short time. However, the examination period is comparatively significantly shorter than previous attempts at measuring cognitive fatigability.

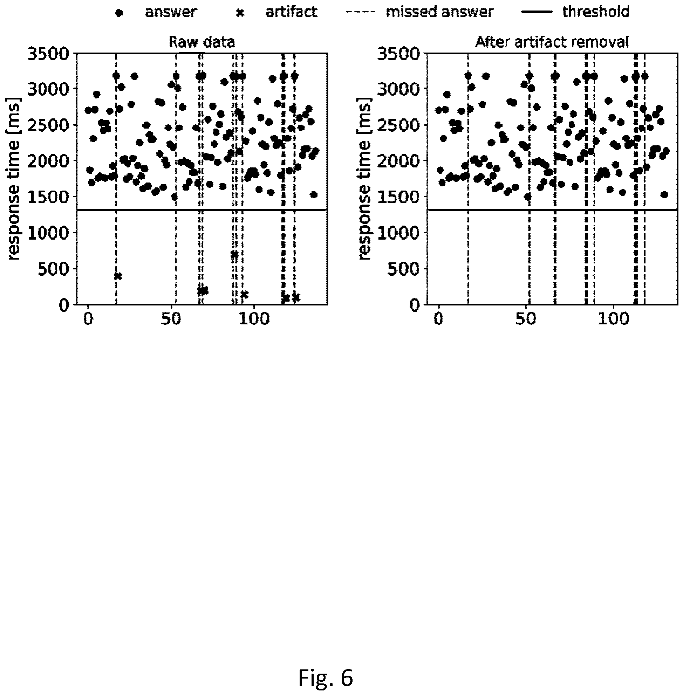

Figs. 6 to 11 depict data measured using the method for determining a fatigue value of a person according to the invention. For the data, 48 persons were recruited from the MS outpatient clinic of the Department of Neurology, University Hospital Zurich, between September 2020 and April 2021. The persons provided written consent following the Declaration of Helsinki The EDSS was obtained from the routine neurologic examinations performed at the hospital. This study was approved by the local ethics committee (Cantonal Ethics Committee Zurich, Switzerland). Inclusion criteria consisted of: (a) confirmed MS diagnosis and (b) age between 18 to 70. In addition, exclusion criteria included: diagnosis of depression, schizophrenia, bipolar disorders, ADHD, and regular intake of psychostimulants or anticonvulsant medications.

The persons were briefly introduced to the study setup and completed a demographic questionnaire. Following, the study examiner showed them the application and the logic of the method according to Fig. 5. The persons started with a two-minute preparation phase. After successful completion, they performed the calibration phase. Next, the persons were asked to complete a first examination phase session with an examination period of 5 minutes which was considered as a trial, to ensure the persons understand the test logic. Following, there was a short break in which the persons filled out an FSMC questionnaire. Next, the persons performed a second examination phase. Previous cognitive fatigability studies including modified versions of the SDMT do full trials and discard this data before conducting the actual test to ensure participants understand the test logic [38]. Hence, all data analyses presented in the following are based on the second examination phase and not on the trial data gained during the first examination phase.

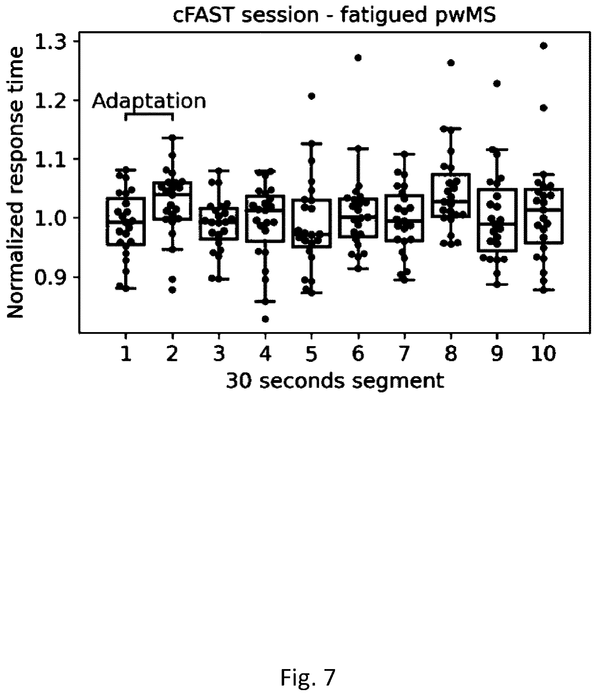

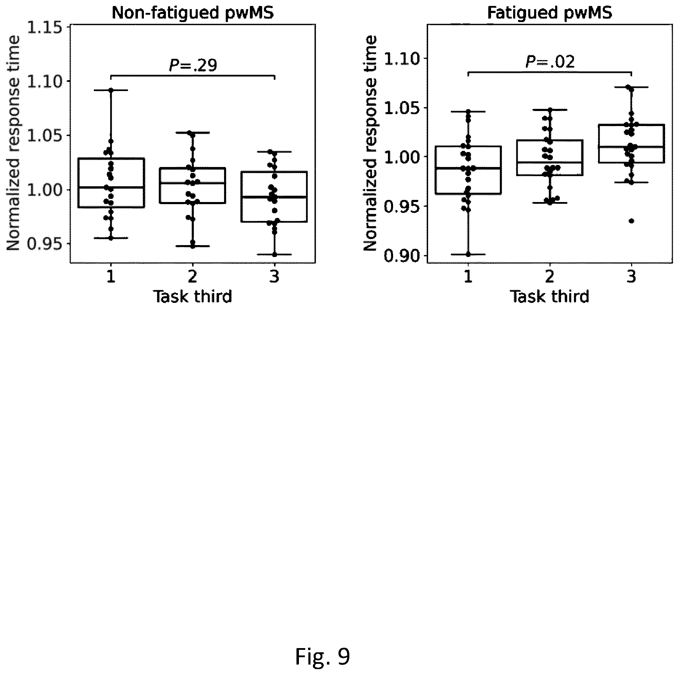

The data were collected from smartphones with touchscreens using a custom Android application. Each sample in the dataset contains the ID of the highlighted symbol 11, the selected symbol 12 if there was any, the respective selection rule 3, and a timestamp of the selection if there was any, otherwise the timestamp of the time limit reset. The data processing pipeline included three steps: (1) artifact detection, cf. Fig. 6; (2) cognitive adaptation removal, cf. Fig. 7; and (3) metrics extraction, cf. for example Table 1, Table 3-5 and Figs. 9-11.

Turning to Fig. 6, the statistical response time was used as one of the primary performance metrics. Artifacts in response time typically appear when a person aims at tapping a symbol of the second group 2 of symbols to match the current highlighted symbol 11, but the person runs out of time and the highlighted symbol 11 and the selection rule 3 are changed while the person is aiming at selecting a symbol based on the previous highlighted symbol and selection rule 3. Hence, the newly displayed highlighted symbol 11 is stored with a short response time and likely with an incorrect answer, and the previous symbol is marked as a missed answer, cf. Fig. 6 left. These artifacts need to be identified and removed to avoid double-counting errors and compute a misleading response time. Therefore, in a preprocessing step, any entry after a missed selection with a response time of less than the average response time determined with the entire examination phase minus two standard deviations of the average response time are removed. This results in subject-specific thresholds that account for the difference in average performance. With this preprocessing, an average of 3.8 entries per session was removed, with the average session containing 138 answers. Fig. 6 right shows the same data after artifact removal.

Previous cognitive fatigue studies describe the existence of an adaptation phase occurring at the beginning of a cognitive task due to some unspecific modulations of training and adaptation and highlight the need to account for these effects when studying fatigue [47,48]. A common strategy to deal with the adaptation in cognitive fatigue studies is to omit the start of the task An adaptation phase is not unique to cognitive tasks as it has also been detected in motor fatigability tasks. A similar strategy is applied in motor tasks by removing the start of the task to account for the adaptation period [25,49,50]. The examination phases exhibited an adaptation period in the initial part of the test, in particular for fatigue patients.

Fig. 7 depicts the average-normalized response times for all fatigued persons with multiple sclerosis for the whole 5 minutes examination period in 30 second sections. During the first sections an increase in response time is observed, followed by a decrease in response time in the third section. These changes in performance are most likely attributed to an adaptation period before persons are fully immersed in the test [47,48]. Hence, to make a fair comparison between the study participants, the first 60 seconds of all examination phases (42 sessions) were discarded before extracting the metrics and performing the data analysis.

Two sets of metrics were used to quantify performance during an examination phase: (1) general metrics, which represent the average performance during an entire examination period, and (2) fatigability metrics, which measure the change in performance occurring between a first and a second section, particularly between the first third and last third of the examination phase. Table 1 displays an overview of the proposed metrics with their definition.

**Table 1: Metrics description**

| Metric name | | description |
|---|---|---|
| **general** | | |
| | *average response time* | average time in milliseconds to tap a digit from the selection panel after displaying a new highlighted symbol |
| | *time limit* | time limit in milliseconds for the displaying of a new highlighted symbol - derived from the calibration phase (corresponds to time limit indicated by the time indicator) |
| | *correct* | total number of selections with a true truth value |
| | *errors* | total number of errors including selections with a false value and no selections before exceeding the time limit |

| **fatigability** | | |
|---|---|---|
| | *Δcorrect* | percent change in *correct* between the first and the last third of the examination period. |
| | *Δresponse time* | percent change in *response time* between the first and the last third of the examination period |
| | *Δerrors* | percent change in *errors* between the first and the last third of the examination period |

Descriptive statistics were used to summarize and compare the study subpopulations. The performance of the derived smartphone-based metrics was evaluated to discriminate between cognitive-fatigued and non-cognitive fatigued subjects following the FSMC cognitive subscale (threshold=22) [7]. With t-tests, group differences were explored, wherein P<.05 was considered significant. Furthermore, through Area Under the Receiver Operating Characteristics (AUROC), the performance of the derived smartphone-based metrics were evaluated to classify cognitive fatigued vs. non-cognitive fatigued subjects, independently of age and EDSS. The robustness of the approach was assessed and confidence intervals for AUROC were calculated using stratified Monte-Carlo sampling [51] with 1000 iterations and by randomly selecting (without replacement) in each iteration 1/2 of the persons' data (examination phases) for evaluation. The data obtained from the examination phases were partitioned into eight strata, following two partitioning criteria: (a) cognitive fatigued as a binary state according to FSMC cognitive subscale (threshold=22) and (b) an EDSS group, which can be one of four: [0,1), [1, 2),[2, 3), and [3,∞). The idea of this partition is to find a metric that works best in the whole spectrum of disability. Each person and their data is fully assigned to one of the resulting eight strata. Thus, when performing the stratified split, either a persons' data is fully contained in the split or not at all. Hence, with this approach, a splitting at the participant level is performed, ensuring for class balance, and accounting for disability. Additionally, as age also influences cognitive performance [31], eight additional strata are created following two partitioning criteria: (a) cognitive fatigued as a binary state and (b) age group, which can be one of four: (18, 30), [30,40), [40,50) and [50, 70]. This partition aims at reducing the influence of age in the metrics by assigning weights according to the group sizes. Furthermore, one-way analysis of covariance (ANCOVA) is used with EDSS as a covariant to rule out the effect of disability when analyzing fatigue. Finally, it is explored how the examination phase and the proposed metrics relate to disability by measuring the performance of the metrics to rate disability according to EDSS. To this end, the study participants were split in two groups according to EDSS and the difference in performance was analyzed between both groups. Patients with EDSS>1.5 were classified as disabled and patients with EDSS<=1.5 were classified as not disabled. For this evaluation, the dataset was partitioned into four strata, following two partitioning criteria: (a) disabled as a binary state according to the EDSS (0 for EDSS<=1.5 and 1 for EDSS>1.5), and (b) cognitive fatigued as a binary state according to FSMC cognitive subscale (threshold=22). Additionally, the same age groups were used as for the cognitive fatigue evaluation. The average AUROC was reported with 95% confidence intervals. In addition, plots of the ROC curves are included for visual inspection.

48 persons were recruited and from those 6 were excluded due to comorbidities including iron deficiency, personality disorder, hypothyroidism, and narcolepsy type 1. Table 2 summarizes the study participants divided into the two subgroups of interest (i.e., no cognitive fatigue and cognitive fatigue according to the FSMC subscore). Of the recruited persons with multiple sclerosis, 21 did not have cognitive fatigue and 27 were cognitively fatigued. Of those included in the analysis, 19 participants did not have fatigue and 23 were fatigued.

**Table 2: Demographic characteristics of participants.**

| | | no fatigue | cognitive fatigue | P |
|---|---|---|---|---|
| Number | | 19 | 23 | |
| Age, mean (SD) gender, n (%) | | 36.89 (12.15) | 38.22 (12.20) | .73 |
| | m | 8 (42) | 6 (26) | .44 |
| | w | 11(58) | 17(74) | |
| MS type, n (%) | | | | |
| | PMS | 1(5) | 3(13) | .61 |
| | RRMS | 18(95) | 20(87) | |
| Disease duration, mean (SD) | | 9.63 (5.88) | 12.52 (8.51) | .20 |
| DMT, n (%) | | | | |
| | None | 1 (5) | 1 (4) | |
| | Interferon beta-1a | 1 (5) | 0 (0) | |
| | Dimethyl fumerate | 2 (11) | 1 (4) | |
| | Teriflunomide | 1 (5) | 1 (4) | |
| | Glatiramer acetate | 1 (5) | 1 (4) | |
| | Fingolimod | 1 (5) | 1 (4) | |
| | Natalizumab | 6 (32) | 8 (35) | |
| | Rituximab | 1 (5) | 3 (13) | |
| | Ocrelizumab | 5 (26) | 7 (31) | |
| Fatigue medication, n (%) | | | | |
| | None | 19(100) | 22 (96) | 1.00 |
| | Modafinil | 0 (0) | 1 (4) | |
| EDSS, mean (SD) | | 1.00 (1.18) | 2.41 (1.95) | .006 |
| FSMC, mean (SD) | | | | |
| | total | 30.84 (8.00) | 64.30 (16.29) | <.001 |
| | cognitive | 14.26 (3.25) | 31.70 (8.81) | <.001 |
| | motor | 16.58 (5.60) | 32.61 (8.68) | <.001 |

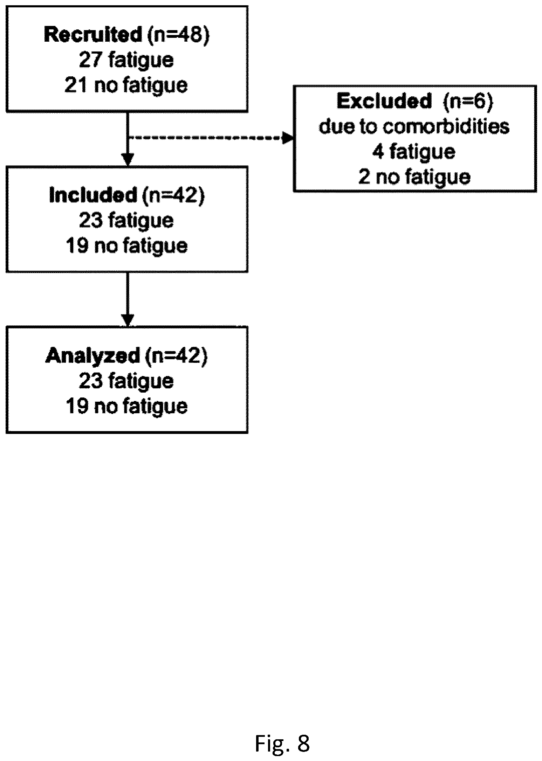

Fig. 8 shows the flow chart of the study and an overview of the excluded patients. The gender distribution of the participants in the two groups, the mean and standard deviation of their age, EDSS, and the FSMC subscales are listed in Table 2. As expected, a significant difference in all the FSMC scores was found. However, no statistically significant difference between the age and gender distributions of the two groups was found.

The analysis indicates a significant Spearman rank correlation between several of the proposed general metrics and the clinical data. Table 3 shows an overview of all the computed correlations. The response time and correct metrics showed the highest correlation with EDSS (p = 0.6, P < .001 and p = -0.6, P < .001, respectively). Then, the time limit follows with p = 0.5, P = .001. On the other hand, errors did not significantly correlate to EDSS (p = -0.07, P = .67). A significant correlation was found when analyzing the relationship between the metrics and the FSMC cognitive subscore. Again, average response time and correct showed the highest correlation to the FSMC subscore (p=0.39, P=.01 and p=-0.38, P=.01, respectively). Neither time limit (p=0.27, P=.09) nor errors (p=0.1, P=.51) significantly correlated to the FSMC cognitive subscore. Age also correlates to the proposed general performance metrics. Among the correlating metrics, correct (p=-0.66, P<.001), response time (p=0.61, P<.001), and time limit (p=0.51, P=.001) was found. No significant correlation was found between the fatigability metrics and the clinical data.

**Table 3: Spearman rank correlation coefficient p: metrics vs. clinical data**

| | EDSS | FSMC cognitive score | age | |
|---|---|---|---|---|
| average response time | 0.6 (<.001) | 0.39 (.01) | 0.61 | (<.001) |
| time limit | 0.5 (.001) | 0.27 (.09) | 0.51 | (.001) |
| correct | -0.6 (<.001) | -0.38 (.01) | -0.66 | (<.001) |
| errors | -0.07 (.67) | 0.1 (.51) | 0.01 | (.93) |
| *Δcorrect* | -0.03 (.86) | -0.21 (.17) | 0.08 | (.59) |
| *Δresponse time* | 0.21 (.17) | 0.24 (.13) | 0.08 | (.59) |
| *Δerrors* | -0.13 (.40) | 0.13 (.42) | -0.2 | (.19) |

The relationship between the metrics and perceived fatigue was investigated by determining statistically significant differences between the cognitive-fatigued and non-fatigued groups.

Table 4 depicts a complete overview of the metrics' mean value and standard deviation for both groups, as well as the t-test results.

A significant difference between both groups was found regarding average response time (t=2.16, P=.04, d=0.669). The group with cognitive fatigue had an average response time of 2586.88 (SD=961.28) ms, compared to the 2083.3 (SD=358.31) ms of non-fatigued participants. No statistically significant difference was found in time limit (t=1.54, P=.13). Furthermore, it was found that correct differed significantly between the groups (t=-2.8, P=.008, d=-0.868). The non-fatigued participants gave an average of 109.11 (SD=15.97) correct answers, while the fatigued group had an average of 90.96 (SD=24.21) correct answers. However, errors was not significantly different between the groups (t=0.29, P=.77).

In terms of the fatigability metrics, it was found that Δresponse time significantly differed between the groups (t=2.27, P=.03, d=0.703). On average, fatigued participants had a Δresponse time of 2.69 (SD=4.94) ms, while non-fatigued participants had an average Δresponse time of -0.96 (SD=5.5) ms. Δerrors and Δcorrect did not show a statistically significant difference between the groups (t=0.81, P=.42 and t=-1.91, P=.06, respectively).

**Table 4: Metrics comparison between fatigued and non-fatigued patients with mean (SD), independent samples t-test (two-tailed) to assess whether there is a statistically significant difference between the groups, and Cohen's d effect size.**

| | | | | | | |
|---|---|---|---|---|---|---|
| | No fatigue | cognitive fatigue | t | P | Cohen's | d |
| Avg. resp. time^{*} | 2083.3 (358.31) | 2586.88 (961.28) | 2.16 | .04 | 0.669 | |
| Time limit^{**} | 3289.47 (1229.75) | 3922.91 (1396.06) | 1.54 | .13 | 0.478 | |
| Correct | 109.11 (15.97) | 90.96 (24.21) | -2.8 | .008 | -0.868 | |
| Errors | 7.58(6.07) | 8.04(4.13) | 0.29 | .77 | 0.091 | |
| Δcorrect | 3.51 (11.19) | -2.73 (9.95) | -1.91 | .06 | -0.593 | |
| Δresponse time | -0.96 (5.5) | 2.69 (4.94) | 2.27 | .03 | 0.703 | |
| Δerrors | -0.46 (2.05) | 0.03 (1.86) | 0.81 .42 | | 0.252 | |

| | | | | | | |
|---|---|---|---|---|---|---|
| ^{*} avg. response time is not normally distributed for the subgroup cognitive fatigue ^{**} time limit is not normally distributed for the subgroups. | | | | | | |

To analyze the temporal progression of participants' performance during an examination phase, a series of paired t-tests was performed. Fig. 9 on the left depicts the average normalized response time in the three thirds of the examination phase for non-fatigued persons with multiple sclerosis and Fig. 9, on the right, shows the results for persons with multiple sclerosis with cognitive fatigue. For the group with no fatigue, the results are primarily flat and with a slight trend to improve over time, while for the fatigued group, a significant increase in response time (P=.02) was found between the first and last third of the session.

The disabled group has a mean EDSS of 3.26 (SD=1.54) and the non-disabled group has a mean EDSS of 0.54 (SD=0.67). Table 5 shows a complete overview of the metrics' average value and standard deviation for both groups, as well as the t-test results.

A significant difference in response time was found between the groups (t=2.47, P=.02, d=0.844). Persons without disability had an average response time of 2080.23 (SD=317.39) ms, compared to the 2696.61 (SD=1030.37) ms exhibited by the disabled persons with multiple sclerosis. Similarly, time limit was significantly lower for participants without disability (t=2.38, P=.02, d=0.737), with an average of 3211.22 (SD=840.63) ms against the 4151.0 (SD=1658.95) ms of disabled participants. Consequently, correct followed the same trend (t=-3.19, P=.003, d=-0.989). On average, disabled persons with multiple sclerosis provided 88.11 (SD=25.44) correct answers, compared to the higher 108.3 (SD=15.1) of persons without disability. No significant difference was found in errors (t=- 0.17, P=.86).

The same analysis was performed with the fatigability metrics. Δcorrect, Δresponse time and Δerrors showed no statistically significant difference between the not disabled and disabled groups (respectively t=-0.3, P=.77, t=1.98, P=.33 and t=-0.6, P=.55).

**Table 5: Metrics comparison between disabled and not disabled patients with mean (SD), independent samples t-test (two-tailed) to assess whether there is a statistically significant difference between the groups, and Cohen's d effect size.**

| | not disabled (n=23) | disabled (n=19) | t | P | Cohen's | d |
|---|---|---|---|---|---|---|
| avg. resp. time^{*} | 2080.23 (317.39) | 2696.61 (1030.37) | 2.47 | .02 | 0.844 | |
| time limit^{**} | 3211.22 (840.63) | 4151.0 (1658.95) | 2.38 | .02 | 0.737 | |
| correct | 108.3 (15.1) | 88.11 (25.44) | -3.19 | .003 | -0.989 | |
| errors | 7.96(5.69) | 7.68(4.26) | -0.17 | .86 | -0.053 | |
| Δcorrect | 0.55 (10.68) | -0.47 (11.35) | -0.3 | .77 | -0.093 | |
| Δresponse time | 0.29 (5.55) | 1.95 (5.33) | 1.98 | .33 | 0.304 | |
| Δerrors | -0.03 (2.05) | -0.4 (1.83) | -0.6 | .55 | -0.187 | |

| | | | | | | |
|---|---|---|---|---|---|---|
| ^{*} average response time is not normally distributed for the subgroup disabled. Levene's Test P<.05 equal variance not assumed. ^{**} time limit is not normally distributed for the subgroups. | | | | | | |

To further explore the association between cognitive fatigability and perceived fatigue, the predictive power of the metrics to classify cognitive fatigue participants according to the FSMC cognitive subscale was assessed. Table 6 shows the results corresponding to the mean AUROC with its respective confidence intervals. The results indicate that the best features for fatigue independently of the EDSS are the fatigability metrics. Δresponse time had the highest AUROC with 0.74 (95% CI 0.64-0.84). Following, Δcorrect and Δerrors with an average AUROC of 0.72 (95% CI 0.63-0.85) and 0.65 (95% CI 0.53-0.77), respectively. From the general metrics, response time performed the best with a mean AUROC of 0.63 (95% Cl 0.50-0.76). The correct metric had an AUROC of 0.62 (95% Cl 0.50-0.74). Calibrated rate produced an AUROC of 0.59 (95% Cl 0.44-0.74). The errors metric showed an AUROC of 0.58 (95% Cl 0.44-0.72). Age had an average AUROC of 0.58 (95% Cl 0.47-0.69). Lastly, EDSS had an AUROC of 0.53 (95% Cl 0.43-0.63).

**Table 6: AUROC score corresponding for cognitive fatigue classification according to the FSMC cognitive subscale for the proposed metrics (sorted by AUROC in descending order).**

| | Metric name | ↓AUROC | (95% Cl) |
|---|---|---|---|
| fatigability | Δresponse time | 0.74 | (95% Cl 0.64-0.84) |
| fatigability | Δcorrect | 0.72 | (95% Cl 0.63-0.85) |
| fatigability | Δerrors | 0.65 | (95% Cl 0.53-0.77) |
| general | average response time | 0.63 | (95% Cl 0.50-0.76) |
| general | correct | 0.62 | (95% Cl 0.50-0.76) |
| general | time limit | 0.59 | (95% Cl 0.44-0.74) |
| general | errors | 0.58 | (95% Cl 0.44-0.72) |
| demographic | age | 0.58 | (95% Cl 0.47-0.69) |
| demographic | EDSS | 0.53 | (95% Cl 0.43-0.63) |

To evaluate the best metrics obtained from the examination phase to classify disability independently of fatigue, the same analysis as done for the cognitive fatigue was performed. The results suggest that the general metrics are better than the fatigability metrics for disability in terms of AUROC. A complete overview of these results is shown in Table 7. Response time produced an average AUROC of 0.64 (95% Cl 0.50- 0.78), followed by age with an average AUROC of 0.63 (95% Cl 0.53-0.73). Following, correct showed an average AUROC of 0.63 (95% Cl 0.49-0.77). Time limit had an average AUROC of 0.59 (95% Cl 0.43-0.75). Δerrors had a mean AUROC of 0.55 (95% Cl 0.41-0.69). Following, Δcorrect produced an AUROC of 0.52 (95% Cl 0.38-0.66). AUROC of errors for disabled patients was 0.51 (95% Cl 0.38-0.64). Finally, Δresponse time was the worst metric for disability with an average AUROC of 0.50 (95% Cl 0.36-0.64).

**Table 7: AUROC score corresponding to disability classification according to the EDSS split with threshold 1.5 for the proposed metrics (sorted by AUROC in descending order).**

| | Metric name | ↓AUROC (95% Cl) |
|---|---|---|
| general | average response time | 0.64 (95% Cl 0.50-0.78) |
| demographics | age | 0.63 (95% Cl 0.53-0.73) |
| general | correct | 0.63 (95% Cl 0.49-0.77) |
| general | time limit | 0.59 (95% Cl 0.43-0.75) |
| fatigability | Δerrors | 0.55 (95% Cl 0.41-0.69) |
| fatigability | Δcorrect | 0.52 (95% Cl 0.38-0.66) |
| general | errors | 0.51 (95% Cl 0.38-0.64) |
| fatigability | Δresponse time | 0.50 (95% Cl 0.36-0.64) |

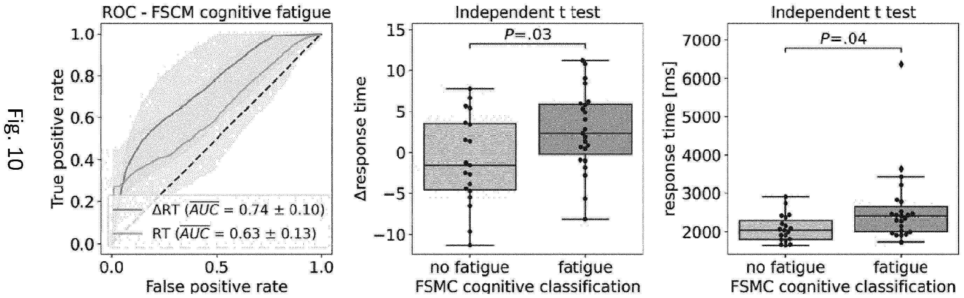

Fig. 10 on the left shows a visual representation of the ROC curves corresponding to the FSMC classification for Δresponse time, best performing feature to classify cognitive fatigue and average response time, best performing feature to classify disability. Δresponse time outperforms average response time by 11 percentage points in classifying fatigue according to the FSMC. The center of the figure shows boxplots of Δresponse time for the groups fatigued and non-fatigued as well as the t-test results. The image displays the statistically significant difference between the fatigue and non-fatigued groups (t=2.27, P=.03). Similarly, the right displays the boxplots corresponding to the average response time. There is a statistically significant difference between the groups (t=2.16, P=.04). The difference is significant also without the outlier in the fatigue group.

A one-way analysis of covariance (ANCOVA) was conducted to examine whether average response time differed between fatigue and non-fatigue groups when controlling for EDSS. For this analysis, the outlier in average response time in the fatigue group was removed as the outlier violated the normality assumptions of ANCOVA. The test assumptions were verified: Shapiro-Wilk test indicates the data is normally distributed for the group with no fatigue W(19)=.926 (P=.15) but not for the fatigued group W(22)=.899 (P=.03). However, as the distribution is close to normal and ANCOVAs are robust to this assumption violation, no steps were taken. Visual analysis with a scatter plot indicates similar regression slopes and an F test indicates no interaction between EDSS and fatigue group F=(1,37)=.24 (P=.64). Finally, Levene's Test confirms the homogeneity of variance with F(1,39)=1.27 (P=.27). ANCOVA analysis reveals that after controlling for EDSS (disability), there was no significant difference in average response time between the fatigue groups F(1,38)=1.42, P=.24.

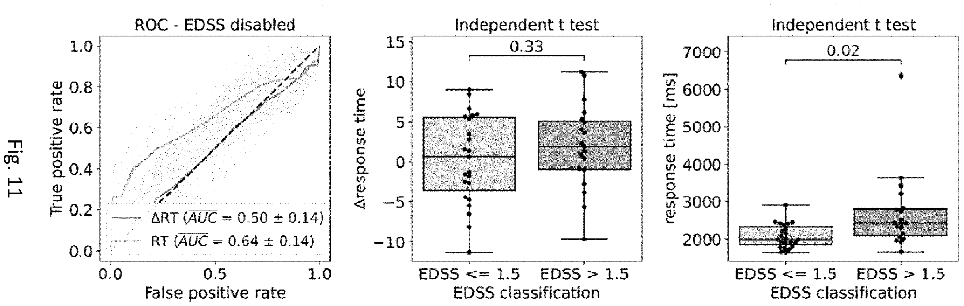

Fig. 11 shows data corresponding to disability classification according to the EDSS threshold. The left side of the Fig. 11 shows a visual representation of the ROC curves corresponding to the disability classification for Δresponse time, the best performing feature to classify cognitive fatigue and response time, the best performing feature to classify disability. In this case, average response time outperforms Δresponse time by 14 percentage points.

With the above, a development process and pilot study of a novel method for determining a fatigue value of a person was described. The results provide early evidence that the invention could be useful to identify patients with cognitive fatigue, as assessed by the FSMC cognitive subscale. So far, only a few studies assess cognitive fatigability with specific tasks for persons with multiple sclerosis [37,38]. Moreover, previous results are contradictory, with some showing fatigability while others not [37,38]. Cognitive fatigability studies are in their infancy, and research could benefit from new approaches and validation studies. The invention differs from previous methods in that it is tailored to patients' disabilities with its calibration mechanism that also enforces rapid decision-making, which contributes to eliciting cognitive fatigability within a single test session and in a short period. In addition, the method may be executed on a smartphone, making it easy to administer, portable, and designed to be applied outside clinical settings, potentially allowing for remote and frequent monitoring. Concerning cognitive testing, healthy controls and persons with multiple sclerosis perceive the PASAT as unpleasant and less likable, while the SDMT is preferred and found appropriate for cognitive testing [44]. Thus, the method according to the invention is likely to find good acceptance as it follows a similar logic to the SDMT and does not require persons to perform arithmetic operations under pressure like the PASAT.

Two sets of metrics were derived within the examination phase: fatigability and general metrics. The initial group level analysis with a t-test revealed statistically significant differences between fatigued and non-fatigued persons with several general and fatigability metrics. Overall, more significant differences were found between the groups with the general metrics than fatigability metrics. However, results from the ANCOVA analysis revealed that EDSS is associated with the metrics response time and correct. Furthermore, the statistical difference in the fatigue groups in terms of these metrics is due to disability and not due to fatigue. Hence, after controlling for EDSS, the statistical difference between the groups disappears. It was further analyzed how the groups' differences related to patients' disabilities. To this end, the study population was divided into two groups according to EDSS, disabled (EDSS>1.5) and non-disabled (EDSS<=1.5). This grouping revealed statistically significant differences with the general metrics but not with the fatigability metrics. This result suggests that general metrics are related to and confounded by disability, while this is not true for the fatigability metrics. The AUROC analysis was conducted controlling for disability with Monte-Carlo simulations and splits were stratified to further rule out the effect of disability from the fatigue analysis. These results confirmed the hypothesis that fatigability metrics are better predictors of fatigue than general metrics. Δresponse time, the best-performing metric to classify fatigue (with an average AUROC of 0.74), is 11 percentage points above average response time, the best-performing general metric for fatigue. Conversely, general metrics dominate the disability classification, with average response time being the best metric (average AUROC of 0.64), 9 percentage points above the best fatigability metric Δerrors. Analysis of the fatigability metrics revealed that, on average, performance during the tests tends to worsen for fatigued patients, while patients without fatigue tend to improve. Previous work on fatigability showed decline towards the end of sustained cognitive activity in pwMS while controls did not [18,42]. The findings go in line with these results. The analysis focused only on persons with multiple sclerosis to decrease disease-specific confoundings.

The invention may allow for remote monitoring. Hence, the method according to the invention seeks to be self-explanatory. For instance, during the preparation phase which aims at familiarizing the persons with the core test logic of selecting symbols according to the selection rule 3. Thanks to the feedback displayed after every selection by means of said signal 4, the persons can quickly realize when they are making mistakes. The immediate feedback, together with the requirement of at least 70% correct answers out of a minimum of 20, helps to assess if the person has correctly understood the test logic and the requirement to perform it quickly. As described above, a predetermined time or time limit may be determined within the calibration phase. The speed requirement seeks to induce cognitive fatigability in a short period. The time limit is derived for each patient, personalizing the method and adjusting for different disability spectra and baseline performance of the patients.

Summarizing, the invention provides a novel method to quantify cognitive fatigability tailored to the persons' disability by said calibration phase. As such, the method may contribute to an objective surrogate of fatigue that allows monitoring of individual patients over time in uncontrolled environments (e.g., at home). Results from the above pilot study provide evidence supporting the validity of the approach and show that the fatigability metrics could potentially be used as a surrogate for perceived cognitive fatigue and motivate further research in this area.

### References

[1] Krupp LB, Alvarez LA, LaRocca NG, et al. Fatigue in multiple sclerosis. Arch Neurol 1988;45: 435-437.
[2] Induruwa I, Constantinescu CS, Gran B. Fatigue in multiple sclerosis - a brief review. J Neurol Sci 2012; 323: 9-15.
[3] Kobelt G, Thompson A, Berg J, et al. New insights into the burden and costs of multiple sclerosis in Europe. Mult Scler 2017; 23: 1123-1136.
[4] Krupp LB, McLinskey N, MacAllister WS. Fatigue in multiple sclerosis. Multiple Sclerosis Therapeutics 2007; 805-818.
[5] Valko PO, Bassetti CL, Bloch KE, et al. Validation of the fatigue severity scale in a Swiss cohort. Sleep 2008; 31: 1601-1607.
[6] Téllez N, Rio J, Tintoré M, et al. Does the Modified Fatigue Impact Scale offer a more comprehensive assessment of fatigue in MS? Mult Scler 2005; 11: 198-202.
[7] Penner IK, Raselli C, Stocklin M, et al. The Fatigue Scale for Motor and Cognitive Functions (FSMC): validation of a new instrument to assess multiple sclerosis-related fatigue. Mult Scler 2009; 15: 1509-1517.
[8] Penner I-K, Paul F. Fatigue as a symptom or comorbidity of neurological diseases. Nat Rev Neurol 2017; 13: 662-675.
[9] Brioschi A, Gramigna S, Werth E, et al. Effect of modafinil on subjective fatigue in multiple sclerosis and stroke patients. Eur Neurol 2009; 62: 243-249.
[10] The Canadian MS Research Group. A Randomized Controlled Trial of Amantadine in Fatigue Associated With Multiple Sclerosis. Can J Neurol Sci 1987; 14: 273-278.
[11] Krupp LB, Coyle PK, Doscher C, et al. Fatigue therapy in multiple sclerosis: results of a double-blind, randomized, parallel trial of amantadine, pemoline, and placebo. Neurology 1995; 45: 1956-1961.
[12] Lange R, Volkmer M, Heesen C, et al. Modafinil effects in multiple sclerosis patients with fatigue. J Neurol 2009; 256: 645-650.
[13] Moller F, Poettgen J, Broemel F, et al. HAGIL (Hamburg Vigil Study): a randomized placebo-controlled double-blind study with modafinil for treatment of fatigue in patients with multiple sclerosis. Mult Scler 2011; 17: 1002-1009.
[14] Nourbakhsh B, Revirajan N, Morris B, et al. Safety and efficacy of amantadine, modafinil, and methylphenidate for fatigue in multiple sclerosis: a randomised, placebo-controlled, crossover, double-blind trial. The Lancet Neurology 2021; 20: 38-48.
[15] Rammohan KW, Rosenberg JH, Lynn DJ, et al. Efficacy and safety of modafinil (Provigil) for the treatment of fatigue in multiple sclerosis: a two centre phase 2 study. J Neurol Neurosurg Psychiatry 2002; 72: 179-183.
[16] Stankoff B, Waubant E, Confavreux C, et al. Modafinil for fatigue in MS: a randomized placebo-controlled double-blind study. Neurology 2005; 64: 1139-1143.
[17] Kluger BM, Krupp LB, Enoka RM. Fatigue and fatigability in neurologic illnesses: proposal for a unified taxonomy. Neurology 2013; 80: 409-416.
[18] Schwid SR, Tyler CM, Scheid EA, et al. Cognitive fatigue during a test requiring sustained attention: a pilot study. Mult Scler 2003; 9: 503-508.
[19] Krupp LB, Elkins LE. Fatigue and declines in cognitive functioning in multiple sclerosis. Neurology 2000; 55: 934-939.
[20] Walker LAS, Lindsay-Brown AP, Berard JA. Cognitive Fatigability Interventions in Neurological Conditions: A Systematic Review. Neurol Ther 2019; 8: 251-271.
[21] Dobkin BH. Fatigue versus activity-dependent fatigability in patients with central or peripheral motor impairments. Neurorehabil Neural Repair 2008; 22: 105-110.
[22] Steens A, de Vries A, Hemmen J, et al. Fatigue perceived by multiple sclerosis patients is associated with muscle fatigue. Neurorehabil Neural Repair 2012; 26: 48-57.
[23] Loy BD, Taylor RL, Fling BW, et al. Relationship between perceived fatigue and performance fatigability in people with multiple sclerosis: A systematic review and metaanalysis. J Psychosom Res 2017; 100: 1-7.
[24] Wolkorte R, Heersema DJ, Zijdewind I. Muscle Fatigability During a Sustained Index Finger Abduction and Depression Scores Are Associated With Perceived Fatigue in Patients With Relapsing-Remitting Multiple Sclerosis. Neurorehabil Neural Repair 2015; 29: 796-802.
[25] Barrios L, Oldrati P, Hilty M, et al. Smartphone-Based Tapping Frequency as a Surrogate for Perceived Fatigue: An in-the-Wild Feasibility Study in Multiple Sclerosis Patients. Proc ACM Interact Mob Wearable Ubiquitous Technol 2021; 5: 1-30.
[26] Walker LAS, Berard JA, Berrigan LI, et al. Detecting cognitive fatigue in multiple sclerosis: method matters. J Neurol Sci 2012; 316: 86-92.
[27] Morrow SA, Rosehart H, Johnson AM. Diagnosis and quantification of cognitive fatigue in multiple sclerosis. Cogn Behav Neurol 2015; 28: 27-32.
[28] Berard JA, Smith AM, Walker LAS. A Longitudinal Evaluation of Cognitive Fatigue on a Task of Sustained Attention in Early Relapsing-Remitting Multiple Sclerosis. International Journal of MS Care 2018; 20: 55-61.
[29] Berard JA, Fang Z, Walker LAS, et al. Imaging cognitive fatigability in multiple sclerosis: objective quantification of cerebral blood flow during a task of sustained attention using ASL perfusion fMRI. Brain Imaging Behav 2020; 14: 2417-2428.
[30] van der Linden D, Frese M, Meijman TF. Mental fatigue and the control of cognitive processes: effects on perseveration and planning. Acta Psychol 2003; 113: 45-65.
[31] Moller MC, Nygren de Boussard C, Oldenburg C, et al. An investigation of attention, executive, and psychomotor aspects of cognitive fatigability. J Clin Exp Neuropsychol 2014; 36: 716-729.
[32] Wang C, Ding M, Kluger BM. Change in intraindividual variability over time as a key metric for defining performance-based cognitive fatigability. Brain Cogn 2014; 85: 251-258.
[33] Burke SE, Babu Henry Samuel I, Zhao Q, et al. Task-Based Cognitive Fatigability for Older Adults and Validation of Mental Fatigability Subscore of Pittsburgh Fatigability Scale. Front Aging Neurosci 2018; 10: 327.
[34] Tombaugh TN. A comprehensive review of the Paced Auditory Serial Addition Test (PASAT). Arch Clin Neuropsychol 2006; 21: 53-76.
[35] Basner M, Dinges DF. Maximizing sensitivity of the psychomotor vigilance test (PVT) to sleep loss. Sleep 2011; 34: 581-591.
[36] Stroop JR. Studies of interference in serial verbal reactions. J Exp Psychol 1935; 18: 643-662.
[37] DeLuca J, Genova HM, Hillary FG, et al. Neural correlates of cognitive fatigue in multiple sclerosis using functional MRI. J Neurol Sci 2008; 270: 28-39.
[38] Chen MH, Wylie GR, Sandroff BM, et al. Neural mechanisms underlying state mental fatigue in multiple sclerosis: a pilot study. J Neurol 2020; 267: 2372-2382.
[39] Pattyn N, Neyt X, Henderickx D, et al. Psychophysiological investigation of vigilance decrement: boredom or cognitive fatigue? Physiol Behav 2008; 93: 369-378.
[40] Agyemang C, Berard JA, Walker LAS. Cognitive fatigability in multiple sclerosis: How does performance decline over time on the Paced Auditory Serial Addition Test? Mult Scler Relat Disord 2021; 54: 103130.
[41] Berard JA, Walker LAS. Increasing the Clinical Utility of the Paced Auditory Serial Addition Test: Normative Data for Standard, Dyad, and Cognitive Fatigability Scoring. Cogn Behav Neurol 2021; 34: 107.
[42] Bryant D, Chiaravalloti ND, DeLuca J. Objective Measurement of Cognitive Fatigue in Multiple Sclerosis. Rehabil Psychol 2004; 49: 114-122.
[43] Fisk JD, Archibald CJ. Limitations of the Paced Auditory Serial Addition Test as a measure of working memory in patients with multiple sclerosis. J Int Neuropsychol Soc 2001; 7: 363-372.
[44] Walker LAS, Cheng A, Berard J, et al. Tests of information processing speed: what do people with multiple sclerosis think about them? Int J MS Care 2012; 14: 92-99.
[45] Pucci E, Branãs P, D'Amico R, et al. Amantadine for fatigue in multiple sclerosis. Cochrane Database Syst Rev 2007; CD002818.
[46] Sheng P, Hou L, Wang X, et al. Efficacy of modafinil on fatigue and excessive daytime sleepiness associated with neurological disorders: a systematic review and metaanalysis. PloS One 2013; 8: e81802.
[47] Möckel T, Beste C, Wascher E. The Effects of Time on Task in Response Selection-An ERP Study of Mental Fatigue. Sci Rep 2015; 5: 10113.
[48] Wascher E, Rasch B, Sanger J, et al. Frontal theta activity reflects distinct aspects of mental fatigue. Biol Psychol 2014; 96: 57-65.
[49] Schwid SR, Thornton CA, Pandya S, et al. Quantitative assessment of motor fatigue and strength in MS. Neurology 1999; 53: 743-750.
[50] Barrios L, Oldrati P, Lindlbauer D, et al. A Rapid Tapping Task on Commodity Smartphones to Assess Motor Fatigability. In: Proceedings of the 2020 CHI Conference on Human Factors in Computing Systems. New York, NY, USA: Association for Computing Machinery, 2020, pp. 1-10.
[51] Preacher KJ, Selig JP. Advantages of Monte Carlo Confidence Intervals for Indirect Effects. Communication Methods and Measures 2012; 6: 77-98.

**List of reference signs**

| | |
|---|---|
| First group | 1 |
| Second group | 2 |
| Selection rule | 3 |
| Signal | 4 |
| Display | 5 |
| User interface | 6 |
| Selection panel | 7 |
| Timer panel | 8 |
| Time indicator | 9 |
| Exit button | 10 |
| Highlighted symbol | 11 |
| Selected symbol | 12 |

## Claims

1. A method, particularly a computer-implemented method for determining a fatigue value of a person, the method comprising the steps of:
i) displaying of:
a) a first group (1) of symbols and a second group (2) of symbols,
b) a selection rule (3) associating each of the symbols of the first group (1) of symbols to one symbol of the second group (2) of symbols,
ii) highlighting one symbol (11) of the first group (1) of symbols,
iii) receiving a user input from the person selecting one symbol (12) of the second group (2) of symbols,
iv) determining a truth value based on the selected symbol (12) of the second group (2) of symbols, wherein the truth value assumes a true value if the selected symbol (12) is selected in accordance with the selection rule (3) and wherein the truth value takes on a false value if not,
v) storing the truth value on a non-transitory memory device,
vi) changing the selection rule (3), such that each of the symbols of the first group (1) of symbols is associated anew to one symbol of the second group (2) of symbols,
vii) changing the highlighted symbol (11) of the first group (1) of symbols,
viii) repeating steps iii) to vii) at least once,
ix) based on at least some of the stored truth values, determining a fatigue value of the person.

2. The method according to claim 1, wherein the highlighted symbol (11) of the first group (1) of symbols is highlighted for a predetermined time, particularly a predetermined time based on a statistical response time of the person, and wherein if the user input is not received within the predetermined time in step iv), the method is set forth with step v) having the truth value set to the false value.

3. The method according to claim 2, wherein during an examination phase, the selection of symbols of the second group (2) of symbols by the person is restricted to a predetermined examination period having a first and a second section, wherein the first section is spaced apart in time from the second section.

4. The method according to claim 3, wherein during the examination phase, the fatigue value is computed by means of a relative change between a first number of stored true and/or false values determined within the first section of the predetermined examination period and a second number of stored true and/or false values determined within the second section of the predetermined examination period.

5. The method according to claim 3 or 4, wherein during the examination phase, the fatigue value is computed by means of a relative change between a first statistical response time determined within the first section of the predetermined examination period and a second statistical response time determined within the second section of the predetermined examination period.

6. The method according to one of the claims 4 to 5, **characterized in that** prior to the examination phase, a preparation phase is conducted, wherein during the preparation phase, steps i) to viii) are executed, wherein upon every selection of a symbol of the second group (2) of symbols by the person, a signal (4) indicative of the truth value of the selected symbol (12) is displayed to the person.

7. The method according to claim 6, wherein the selection of symbols of the second group (2) of symbols by the person is restricted to a predetermined preparation period of the preparation phase.

8. The method according to claim 7, **characterized in that** between the preparation phase and the examination phase, a calibration phase is conducted, wherein during the calibration phase:
- steps i) to viii) are executed,
- the selection of symbols of the second group (2) of symbols by the person is restricted to a predetermined calibration period and
- the statistical response time is determined, wherein the statistical response time is determined from time intervals between the displaying of a highlighted symbol (11) of the first group (1) of symbols and the selection of a symbol of the second group (2) of symbols by the person.

9. The method according to claim 8, wherein the method according to claim 8 is only performed if during the method according to claim 7, the person has selected a predetermined minimum number of symbols of the second group (2) of symbols and if a predetermined minimum ratio between
- a number of true values determined based on the selected symbols (12) and
- the minimum number of symbols
has been determined.

10. A computer program comprising instructions which, when the program is executed by a processing unit, cause the processing unit to carry out the steps i) to ix) of the method according to one of the preceding claims.

11. A system for determining a fatigue value of a person, wherein the system comprises:
- a display (5) configured to display:
a) a first group (1) of symbols and a second group (2) of symbols,
b) a selection rule (3) logically associating each of the symbols of the first group (1) of symbols to a respective associated symbol of the second group (2) of symbols,
wherein one symbol (11) of the first group of symbols is highlighted,
- a user interface (6) for receiving a user input from the person, wherein the user input is indicative of selections of symbols of the second group (2) of symbols by the person and
- a processing unit, particularly configured to execute the computer program of claim 10, wherein the processing unit is configured to
i) determine truth values based on the selected symbols (12) of the second group (2) of symbols, wherein the truth value assumes a true value if the selected symbol (12) of the second group (2) of symbols was selected in accordance with the selection rule (3) and wherein the truth value assumes a false value if not
ii) store the truth values on a non-transitory memory device
iii) change the selection rule (3) upon every selection of a symbol of the second group (2) of symbols by the person such that each of the symbols of the first group (1) of symbols is associated anew to one symbol of the second group (2) of symbols,
iv) change the highlighted symbol (11) of the first group (1) of symbols upon every selection of a symbol of the second group (2) of symbols by the person and
v) determine a fatigue value of the person based on at least some of the stored truth values.

12. The system according to claim 11, wherein the system is configured to set a time limit for the person to select a symbol of the second group (2) of symbols and to cause said display (5) to display a time indicator (9) indicative of a time left until said time limit is reached and wherein the system is further configured to change said highlighted symbol (11) of the first group (1) of symbols and said selection rule (3) if the person does not select a symbol of the second group (2) of symbols within said time limit.

13. The system according to claim 12, wherein the system is further configured to set the truth value to the false value if the person does not select a symbol of the second group (2) of symbols within the time limit.

14. The system according to one of the claims 11 or 13, wherein the processing unit is configured to set a predetermined examination period for selection of symbols of the second group (2) of symbols by the person and wherein the processing unit is further configured to determine and particularly to output the fatigue value based on a relative change of a first number of true or false values determined within a first section of the predetermined examination period and a second number of true or false values determined within a second section of the predetermined examination period, wherein the second section is spaced apart in time from the first section; and/or based on a relative change of a first statistical response time determined within the first section of the predetermined examination period and a second statistical response time determined within the second section of the predetermined examination period.

15. The system according to claim 12, wherein the system is further configured to determine a statistical response time of the person determined from time intervals between the displaying of a highlighted symbol (11) of the first group (1) of symbols and the selection of a symbol of the second group (2) of symbols by the person, wherein the time limit is based on the statistical response time of the person.
